# EUROPÄISCHE PATENTSCHRIFT

(11) **EP 1 162 995 B1**
(45) Veröffentlichungstag und Bekanntmachung des Hinweises auf die Patenterteilung: **04.06.2003**
(21) Anmeldenummer: 00925114.1
(22) Anmeldetag: 15.03.2000
(51) Int. Cl.: A61K 38/46, A61P 3/10

(54) **ENZYME ZUR BEHANDLUNG VON DIABETES MELLITUS TYP I**
ENZYMES FOR THE TREATMENT OF DIABETES MELLITUS TYPE I
ENZYMES POUR LE TRAITEMENT DU DIABETE MELLITUS DE TYPE I

(30) Priorität: 17.03.1999 DE 19911778
(43) Veröffentlichungstag der Anmeldung: 19.12.2001
(73) Patentinhaber: Solvay Pharmaceuticals GmbH, 30173 Hannover (DE)
(72) Erfinder: SANDER, Suntje, D-30173 Hannover (DE); STEINBORN, Claus, D-30926 Seelze (DE); RUDMANN, Martin, D-29339 Wathlingen (DE); SCHWANITZ, Dieter, D-31036 Eime (DE); HENNIGES, Friederike, D-38102 Braunschweig (DE)
(74) Vertreter: Gosmann, Martin
(86) Internationale Anmeldenummer: EP0002261
(87) Internationale Veröffentlichungsnummer: WO00054799

(56) Entgegenhaltungen:
- US-A- 3 803 305
- DELHAYE M. ET AL: "Comparative evaluation of a high lipase pancreatic enzyme preparation and a standard pancreatic supplement for treating exocrine pancreatic insufficiency in chronic pancreatitis." EUROPEAN JOURNAL OF GASTROENTEROLOGY AND HEPATOLOGY, (1996) 8/7 (699-703). , XP000953004
- KLOTZ H P: "[Letter: Lyophilized pancreatic extract, a therapeutic adjuvant of light diabetes ]. L'extrait pancreatique lyophilise, adjuvant therapeutique du diab ete leger." NOUVELLE PRESSE MEDICALE, (1975 OCT 4) 4 (32) 2333. , XP000952822
- DATABASE MEDLINE [Online] US NATIONAL LIBRARY OF MEDICINE (NLM), BETHESDA, MD, US; SIMEK I ET AL: "[Substitution therapy in insufficient external pancreatic secretion]. Substitucni lecba insuficience zevni sekrece pankreatu." retrieved from STN Database accession no. 93282167 XP002150548 & VNITRNI LEKARSTVI, (1993 MAR) 39 (3) 250-2. ,
- D'COSTA D.F. ET AL: "Diabetic neuropathic cachexia associated with malabsorption." DIABETIC MEDICINE, (1992) 9/2 (203-205). , XP000946992

## Beschreibung

Die vorliegende Erfindung betrifft die Verwendung von physiologisch akzeptablen Enzymgemischen mit lipolytischer, proteolytischer und amylolytischer Aktivität, insbesondere aber von Verdauungsenzymgemischen wie z.B. Pankreatin zur Herstellung von pharmazeutischen Zubereitungen zur Behandlung von primären Diabetes mellitus Type I, und zur Herstellung von für diese Behandlung geeigneten Arzneimitteln. Insbesondere betrifft die Erfindung hierbei die Verwendung dieser Enzymgemische mit lipolytischer, proteolytischer und amylolytischer Aktivität, insbesondere aber von Pankreatin oder pankreatinhaltigen Verdauungsenzymgemischen zur adjuvanten Behandlung von primärem Diabetes mellitus Typ I.

Unter dem Begriff Diabetes wird im allgemeinen Diabetes mellitus, die sogenannte "Zuckerkrankheit", verstanden. Man unterscheidet hierbei - neben anderen, z.B. sekundären Diabetesformen, die als Folgeerkrankungen anderer Primärerkrankungen auftreten können - im wesentlichen zwei Hauptgruppen von Störungen des Kohlenhydratstoffwechsels, nämlich Diabetes Typ I infolge Insulinmangels und Diabetes Typ II infolge verminderter Insulinwirkung, wobei der Verlauf der Erkrankung u.a. vom jeweiligen Typ abhängt. Diabetes ist weiterhin eine chronische Erkrankung mit verschiedenartigen pathologischen Erscheinungsbildern und wird z.B. von Störungen des Fettstoffwechsels, des Kreislaufs wie auch des Glukosestoffwechsels begleitet. Zu den typischen Symptomen dieser Erkrankung zählen erhöhter Blutzucker (Hyperglykämie), Zuckerausscheidung im Harn (Glukosurie), Neigung zu Infekten und Juckreiz. Diabetes tendiert zur Progression und bringt darüber hinaus in vielen Fällen verschiedene Komplikationen mit sich. Als Komplikationen sind z.B. Nerven- und Blutgefäßerkrankungen bekannt. Es ist daher notwendig, in jeder Phase der Erkrankung die Therapie an jeden individuellen Fall anzupassen und die für den individuellen Fall geeigneten Arzneimittel auszuwählen. Darüber hinaus kann es im Rahmen dieser Therapie wünschenswert sein, neben dem primär ausgewählten Arzneimittel zusätzlich weitere Arzneimittel im Rahmen einer adjuvanten Behandlung heranzuziehen, die einen unterstützenden Effekt auf die Therapie bzw. einen positiven Einfluß auf den weiteren Verlauf der Erkrankung ausüben können.

Das Dokument US 3,803,305 offenbart die Verwendung von pankreatischen Extrakten zur Behandlung von nicht-insulinabhängigem Diabetes. In dem Dokument von Klotz [Klotz H.P. L'extrait pancréatique lyophilisé, adjuvant thérapeutique du diabète léger; Nouvelle Presse Médicale (1975) 4(32): 2333] wird die hypoglykämische Wirkung eines pankreatischen Extraktes in Patienten mit leichtem Diabetes beschrieben; der Autor nimmt an, dass der pankreatische Extrakt durch Senkung der Plasmalipide und durch Steigerung der Wirksamkeit des endogenen Insulins wirkt.

Der Erfindung liegt somit die Aufgabe zugrunde, neue pharmazeutische Zubereitungen zur Behandlun von bestimmten Formen des Diabetes mellitus bereitzustellen. Insbesondere liegt der Erfindung die Aufgabe zugrunde, neue pharmazeutische Zubereitungen zur adjuvanten Behandlung in der Diabetes-Therapie bereitzustellen, die einen zusätzlichen unterstützenden Effekt auf die Therapie bzw. einen positiven Einfluß auf den weiteren Verlauf der diabetischen Erkrankung, z.B. durch Verminderung von Spätfolgen, ausüben.

Erfindungsgemäß werden nun zur Herstellung von pharmazeutischen Zubereitungen zur Behandlung von Primären Diabetes mellitus Typ I in größeren Säugetieren und Menschen physiologisch akzeptable Enzymgemische mit lipolytischer, proteolytischer und amylolytischer Aktivität, wie beispielsweise entsprechende Enzymgemische mikrobieller Herkunft und/oder insbesondere tierische Verdauungsenzymgemische wie vorzugsweise Pankreatin oder Pankreatin-ähnliche Verdauungsenzymgemische, verwendet.

Im Rahmen der vorliegenden Erfindung können dabei physiologisch akzeptable Enzymgemische mit lipolytischer, proteolytischer und amylolytischer Aktivität eingesetzt werden, die beliebigen tierischen oder mikrobiologischen Ursprungs sind. Die im Rahmen der Erfindung eingesetzten Enzymgemische mit lipolytischer, proteolytischer und amylolytischer Aktivi-tät können daher sowohl rein mikrobiellen Ursprungs, rein tierischen Ursprungs oder auch ein Gemisch von Enzymen tierischen und mikrobiellen Ursprungs darstellen.

In einer Variante der Erfindung ist daher das eingesetzte Enzymgemisch rein mikrobiellen Ursprungs. Als mikrobielle Enzyme kommen insbesondere Enzyme aus Bakterien, wie z.B. aus den Stämmen Bacillus oder Pseudomonas, oder aus Pilzkulturen wie Schimmelpilzen, beispielsweise des Stammes Rhizopus oder Aspergillus, in Frage. Beispiele für solche physiologisch akzeptablen bakteriellen und/oder Schimmelpilz-Enzyme sind bereits im Stand der Technik beschrieben, z.B. im Zusammenhang mit ihrer Gewinnung und Verwendung zur Behandlung von Maldigestion. Lipasen können hierbei z.B. aus Bacillus- oder Pseudomonas-Stämmen, Amylasen und auch Lipasen aus Schimmelpilzen, beispielsweise des Stammes Rhizopus, und Proteasen z.B. ebenfalls aus Aspergillus entstammen.

Insbesondere wird es sich im Rahmen der Erfindung aber in einer bevorzugten Variante um solche Verdauungsenzymgemische mit lipolytischer, proteolytischer und amylolytischer Aktivität handeln, die in ihren Eigenschaften dem Pankreatin nahestehen. Bevorzugt sind daher im Rahmen der vorliegenden Erfindung pankreatinhaltige Verdauungsenzymgemische sowie insbesondere Pankreatin selbst, wobei dem Pankreatin oder den pankreatinhaltigen Verdauungsenzymgemischen aber gewünschtenfalls noch ein oder mehrere mikrobielle, d.h. aus Mikroorganismen gewonnene Enzyme aus der Gruppe der Lipasen, Proteasen und Amylasen zugesetzt sein können.

Pankreatin ist ein bekanntes Enzymgemisch mit lipolytischer, proteolytischer und amylolytischer Aktivität, welches z.B. unter dem Markennamen Kreon® in Form von Granulaten, Pellets oder Kapseln mit magensaftresistenten Mikropellets im Handel erhältlich ist und medizinisch zur Enzymsubstitution z.B. bei Pankreasinsuftizienz, Verdauungsinsuffizienz nach Magenoperationen, Leber- und Gallenerkrankungen, Mucoviszidose und chronischer Pankreatitis eingesetzt wird. Pankreatin wird im allgemeinen als natürliches Enzymgemisch durch Extraktion aus Schweinepankreas gewonnen, z.B. nach den in den deutschen Patentanmeldungen DE 25 12 746 und DE 42 03 315 beschriebenen Verfahren, und nachfolgend in an sich bekannter Weise in die gewünschte galenische Form überführt. Die Pankreasenzyme werden üblicherweise in Form fester Zubereitungen oral verabreicht.

Die erfindungsgemäß hergestellten pharmazeutischen Zubereitungen enthalten in einer Variante der Erfindung vorzugsweise Pankreatin oder pankreatinhaltige Verdauungsenzymgemische. In diesen erfindungsgemäß hergestellten pharmazeutischen Zubereitungen können Pankreatin oder pankreatinhaltige Verdauungsenzymgemische gewünschtenfalls neben Pankreatin zusätzlich noch ein oder mehrere physiologisch akzeptable Enzyme aus der Gruppe der Lipasen, Proteasen und Amylasen enthalten, wie diese aus Mikroorganismen gewonnen werden können. Als mikrobielle Enzyme kommen für diesen Zusatz insbesondere die oben bereits genannten Enzyme aus Bakterien, wie z.B. aus den Stämmen Bacillus oder Pseudomnas, oder aus Pilzkulturen wie Schimmelpilzen, beispielsweise des Stammes Rhizopus oder Aspergillus, in Frage. Die dem Pankreatin oder den pankreatinhaltigen Enzymgemischen zugesetzten Lipasen können hierbei z.B. aus Bacillus- oder Pseudomonas-Stämmen, zugesetzte Amylasen und auch Lipasen aus Schimmelpilzen, beispielsweise des Stammes Rhizopus, und zugesetzte Proteasen z.B. ebenfalls aus Aspergillus entstammen.

Es wurde nun gefunden, daß die im Rahmen dieser Erfindung beschriebenen, physiologisch akzeptablen Enzymgemische mit lipolytischer, proteolytischer und amylolytischer Aktivität, die mikrobiellen und/oder tierischen Quellen entstammen können, nicht nur zur Behandlung von Verdauungsenzym-Mangelzuständen - wie diese beispielsweise durch krankhafte Veränderung des Pankreas aufgrund chronischer Pankreatitis, Verdauungsinsuffizienz nach Magenoperationen, Leber- oder Gallenerkrankungen hervorgerufen werden - eingesetzt werden können, sondern sich überraschenderweise auch für die Behandlung von primärem Diabetes mellitus Typ I in größeren Säugetieren und Menschen eignen. Insbesondere aber eignen sich die vorstehenden physiologisch akzeptablen Enzymgemische mit lipolytischer, proteolytischer und amylolytischer Aktivität, vorzugsweise aber z.B. Pankreatin selbst oder pankreatinhaltige Verdauungsenzymgemische, die noch mikrobielle Enzymzusätze, z.B. eine oder mehrere mikrobielle Lipasen, Proteasen und/oder Amylasen enthalten, zur adjuvanten Behandlung in der Therapie des primären Typ I - Diabetes, wobei sie einen zusätzlichen, die Diabetes-Therapie unterstützenden Effekt bzw. einen positiven Einfluß auf den weiteren Verlauf der diabetischen Erkrankung ausüben, d.h. insbesondere zur Verminderung von Spätfolgen der diabetischen Erkrankung beitragen.

Hierbei weist die erfindungsgemäße Verwendung der eingesetzten physiologisch akzeptablen Enzymgemische, vorzugsweise z.B. Pankreatin oder pankreatinhaltige Verdauungsenzymgemische mit gegebenenfalls noch mikrobiellen Enzymzusätzen, Vorteile auch bei solchen Patienten auf, bei denen zum primären Diabetes mellitus Typ I zusätzlich noch Komplikationen in bezug auf eine gleichfalls vorliegende exokrine Pankreasinsuffizienz vorliegen.

Die pharmazeutischen Zubereitungen können neben den beschriebenen mikrobiellen Enzymgemischen und/oder tierischen Verdauungsenzymgemischen, wie insbesondere Pankreatin oder pankreatinhaltige Verdauungsenzymgemische, zusätzlich pharmazeutische Hilfsstoffe und/oder Zusatzstoffe und ggf. Stabilisatoren enthalten.

So können die Enzymgemische in einer wirksamen Menge (jeweils festgelegt anhand der aktiven Einheiten der lipolytischen, proteolytischen und amylolytischen Komponenten) zusammen mit üblichen pharmazeutischen Hilfs- und/oder Trägerstoffen in festen oder flüssigen pharmazeutischen Zubereitungen enthalten sein. Pro Dosierungs-Einheit kann hierbei die lipolytische Akivität im allgemeinen im Bereich von 5000 bis 45000 Ph.Eur. Lipase-Einheiten, die proteolytische Aktivität im allgemeinen im Bereich von 200 bis 3000 Ph.Eur. Protease-Einheiten und die amylolytische Aktivität im allgemeinen im Bereich von 3500 bis 45000 Ph.Eur. Amylase-Einheiten liegen (Ph.Eur. Pharmacopoeia Europea). Als Beispiele für typische Dosierungs-Einheiten seien z.B. Enzymgemische mit folgenden Aktivitäten genannt: a) ca. 10000 Ph.Eur. Lipase/ca. 8000 Ph.Eur. Amylase/ca. 600 Ph.Eur. Protease; b) ca. 25000 Ph.Eur. Lipase/ca. 18000 Ph.Eur.Amylase/ca. 1000 Ph.Eur. Protease; bzw. c) ca. 40000 Ph.Eur. Lipase/ca. 40000 Ph.Eur. Amylase/ca. 2600 Ph.Eur. Protease. Als Beispiele fester Präparate seien oral applizierbare Präparate wie Tabletten, Dragees, Kapseln, Pulver, Granulate oder Pellets genannt. Diese festen Präparate können pharmazeutisch übliche anorganische und/oder organische Trägerstoffe wie z.B. Milchzucker, Talkum oder Stärke neben pharmazeutisch üblichen Hilfsmitteln, beispielsweise Gleitmitteln oder Tablettensprengmitteln, enthalten. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z.B. Konservierungsmittel, Geschmackskorrigenzien, Stabilisatoren (z.B. komplexe Lipide) und dergleichen.

Die Enzymgemische können mit den pharmazeutischen Hilfsund/oder Trägerstoffen in an sich bekannter Weise gemischt und formuliert werden. Zur Herstellung fester Arzneiformen können die Enzymgemische beispielsweise mit den Hilfsund/oder Trägerstoffen in üblicher Weise gemischt und naß oder trocken granuliert oder pelletiert werden. Granulate, Pellets oder Pulver können direkt in Kapseln oder Sachets abgefüllt oder in üblicher Weise zu Tablettenkernen verpreßt werden. Diese können gewünschtenfalls in bekannter Weise dragiert werden. Flüssige Zubereitungen können durch Auflösen oder Dispergieren der Komponenten und ggf. weiterer Hilfsstoffe in einem geeigneten flüssigen Träger, in Form von Lösungen oder Suspensionen erhalten werden.

Die antidiabetischen Effekte sowie der positive Einfuß auf den Verlauf der diabtischen Erkrankung, inbesondere im Rahmen einer adjuvanten Behandlung in einer Diabetes-Therapie, können für die erfindungsgemäß verwendeten physiologisch akzeptablen Enzymgemische mit lipolytischer, proteolytischer und amylolytischer Aktivität, wie z.B. die oben beschriebenen mikrobiellen Enzymgemische und insbesondere Pankreatin oder pankreatinhaltige Verdauungsenzymgemische, durch Bestimmung der Wirkung auf pharmakologische Parameter, wie diese im allgemeinen zur Beurteilung diabetischer Erkrankungen herangezogen werden, nachgewiesen werden. Solche Parameter können z.B. die Verbesserung, d.h. Abnahme des glykosylierten Haemoglobins (HbA_{1c}), Abnahme des Blutglukosespiegels, Verminderung Hypoglykämischer Anfälle sowie Abnahme von Hyperglykämie sein.

Zum Nachweis der positiven Effekte der beschriebenen Enzymgemische auf die glykämische Kontrolle wurde eine doppelt verblindete, multizentrische-, placebokontrollierte Parallelgruppen-Studie mit Panreatin in Form von Kreon® 10000 Minimicro-spheres® (Minimikropellets in Kapseln) an Insulin-abhängigen diabetischen Patienten (IDDM = Typ I) mit zusätzlicher exokriner Pankeasinsuffizienz durchgeführt. Die Patientengruppe (männlich/weiblich) war zufallsverteilt mit einer Anzahl von je etwa 74 Patienten pro Gruppe. Die Behandlung wurde über einen Zeitraum von 4 Monaten durchgeführt mit einer oralen Dosierung von 16 bis 18 Kreon-Kapseln pro Tag. Jeweils 4 Kapseln wurden zu den Hauptmahlzeiten (3 pro Tag) und je 2 Kapseln zu Zwischenmahlzeiten (2 bis 3 pro Tag) verabreicht. Eine Kapsel mit Kreons® 10000 Minimicrospheres® enthielt 150 mg Pankreatin mit angegebenen Enzymgehalten von 10000 Ph.Eur. Lipaseeinheiten, 8000 Ph.Eur. Amylaseeinheiten und 600 Ph.Eur. Proteaseeinheiten. Die Placebo-Gruppe erhielt entsprechende Placebo-Minimicrosphere-Kapseln ohne Enzymaktivitäten.

Die Wirksamkeit des eingesetzten Enzymgemisches in bezug auf die glykämische Kontrolle wurde durch Messung des glykosylierten Haemoglobin-Spiegels (HbA_{1C}) bestimmt. Hierbei ist eine positive Beeinflussung des HbA_{1C}-Spiegels als klinisch relevante Verbesserung des Diabetes mellitus Status erwünscht. Als weitere diabetische Parameter wurden die Blutglukose-Spiegel (Insulin/Glukagon), die Beurteilung hypoglykämischer Attacken, der Status fettlöslicher Vitamine (A, D und E), die tägliche Insulindosis, der Körpergewichtsindex und hyperglykämische Perioden herangezogen.

Nach einer Vorstudien-Bewertung zur Selektion des Patientenkollektivs durchliefen die Patienten eine Anlaufphase von 8 Wochen (ohne Enzymgemisch/Placebo-Verabreichung), um die Patienten auf individuelle Insulindosierungen einzustellen. Vor dem Beginn der eigentlichen doppelblind-Parallelgruppen-Untersuchungsperiode von 4 Monaten wurde dann eine Basislinien-Bewertung vorgenommen. Die Insulindosis sollte während der Studie möglichst stabil bleiben (± 10 %), mit Ausnahme des ersten Behandlungsmonats, in dem noch eine Adjustierung der Insulindosierung erlaubt war. Eine erforderliche Insulin-Adjustierung nach der erfolgten Randomisierung führte zum Aussschluß des Patienten aus der Studie, jedoch waren kurzfristige Anpassungen infolge z.B. akuter Krankheit erlaubt. Die Patienten durchliefen mehrere Zwischenbewertungen im Verlauf der Studie und zum Abschluß wurde eine weitere Bewertung vorgenommen. Getrennt von den diabetischen Parametern wurden auch gastroenterologische Parameter wie Stuhlfett, Stuhlcharakteristik, Fettabsorptionskoeffizient (CFA, Coefficient of Fat Absorption) und klinische Symptome gemessen.

Anhand der vorstehend skizzierten Untersuchung zeigt sich eine positive Beeinflussung der Diabetes-Parameter bei Verabreichung von Pankreatin, sowohl im Hinblick auf eine positive Beeinflussung des glykosylierten Haemoglobins (HbA_{1C}) als auch im Hinblick auf eine verbesserte glykämische Kontrolle: wie z.B. Stabilisierung der Blutzuckerverläufe (Glättung) und z.B. Verminderung von hypoglykämischen Attacken sowie von Hyperglykämie. Die Studienergebnisse zeigen somit, daß durch erfindungsgemäße Verabreichung von physiologisch akzeptablen Enzymgemischen mit lipolytischer, proteolytischer und amylolytischer Aktivität eine verbesserte Einstellung des primären Diabetes mellitus Typ I erreicht werden kann. Es konnte getrennt davon gezeigt werden, daß im diabetischen Patientenkollektiv mit begleitender exokriner Pankreasinsuffizienz die gastroenterologischen Parameter wie z.B. Stuhlfett, Stuhlcharakteristik, CFA und klinische Symptome ebenfalls positiv beeinflußt werden und der Ernährungszustand dieser Patienten insgesamt verbessert wird. Physiologisch akzeptable Enzymgemische mit lipolytischer, proteolytischer und amylolytischer Aktivität - insbesondere Pankreatin oder pankreatinhaltige Enzymgemische, auch mit den beschriebenen mikrobiellen Enzymzusätzen - eignen sich daher zur Herstellung von pharmazeutischen Zubereitungen zur Behandlung von primären Diabetes mellitus Typ I, insbesondere zur adjuvanten Behandlung von primären Diabetes mellitus Typ I, hierbei kann der primäre Diabetes mellitus Typ I auch durch exokrine Pankreasinsuffizienz begleitet sein.

Die pharmakologisch günstigen Wirkungen des eingesetzten Enzymgemisches auf die diabetischen Parameter und die Status-Verbesserung des Diabetes mellitus Typ I soll durch die nachfolgend beschriebene klinische Studie und die dabei erzielten Ergebnisse weiter erläutert werden.

### Klinische Studie

Zur Evaluierung der Wirksamkeit von Kreon® 10000 Minimicrospheres® im Vergleich zu Placebo auf die glykämische Kontrolle von Insulin-abhängigen diabetischen Patienten (IDDM = insulin dependent diabetes mellitus) mit exokriner Pankreasinsuffizienz wurde eine doppelt verblindete, multizentrische, placebokontrollierte Parallelgruppen-Studie durchgeführt.

### I. Studienkonzept

Der nachfolgende klinische Untersuchungsbericht ist eine Interimsauswertung einer Studie zur Wirksamkeit von Kreon® 10000 Minimicrospheres® in der Behandlung von Insulin-abhängigen Patienten (IDDM) durch glykämische Kontrolle. Der Bericht gibt demzufolge einen Überblick über die Studie und über die nach Einbeziehung von etwa der Hälfte der erforderlichen Patienten zwischenzeitlich erzielten Ergebnisse.

### Ziel der Studie

Das Hauptziel der Studie war, die Wirkung von Kreon® 10000 Minimicrospheres® auf die glykämische Kontrolle von Insulinabhängigen Diabetikern mit exokriner Pankreasinsuffizienz zu evaluieren.

Es gibt mehrere Variablen, um die glykämische Kontrolle zu bewerten. Der wichtigste Parameter ist HbA_{1c}, der bei der behandelten Gruppe niedriger als bei der Placebogruppe sein sollte. Dieser Parameter wurde als Hauptparameter gewählt. Als zusätzliche Parameter wurden die Anzahl der hyperglykämischen Attacken und der hyperglykämischen Vorfälle, die Blutzuckerwerte im nüchternem Zustand und nach dem Essen, die tägliche Insulindosis, das Körpergewicht und die Plasmaspiegel von fettlöslichen Vitaminen (A/D und E) untersucht. Weiterhin wurden Parameter der exokrinen Funktionsstörung des Pankreas bewertet, darunter die Frequenz und Konsistenz des Stuhls, abdominale Schmerzen, Meteorismus, Flatulenz und der klinische Gesamteindruck der Krankheit aus Sicht des Patienten und des Untersuchenden.

Tolerierbarkeit und Sicherheit wurden mittels Standardtests bewertet.

### Patienten-Population:

Für diese Studie wurden Patienten mit einem Insulin-abhängigen Diabetes mellitus herangezogen. Diese Patienten-Population wurde als Modell-Testgruppe für Diabetes mellitus Patienten ausgesucht, da diese Gruppe am besten definiert ist. Es war geplant, 74 Patienten pro Behandlungsgruppe einzuschließen, um eine Testgruppengröße von insgesamt 148 Patienten zu erreichen.

Die Patienten sollten einen Krankheitbeginn im Alter vor 30 aufweisen und sollten mit der Insulintherapie innerhalb eines Jahres nach der Diagnose begonnen haben. Patienten mit nachgewiesenem nicht Insulin-abhängigen Diabetes mellitus wurden ausgeschlossen.

Um sicherzustellen, daß die zufallsverteilten Patienten einen bestimmten Grad von exokriner Pankreasinsuffizienz aufwiesen, wurden die faekalen Elastase-1 Werte ausgewertet. Dieser Parameter ist ein Maß zur Feststellung einer vorhandenen exokrinen Fehlfunktion des Pankreas. Für die Teilnahme an der Studie mußte der Elastase-1 Wert kleiner/gleich 100 µg/g Stuhl sein. Patienten mit darüberliegende Werten wurden von der Studie ausgeschlossen. Patienten mit exokriner Pakreasinsuffizienz aus anderen Gründen als Insulin-abhängiger Diabetes mellitus (z.B. chronische Pankreatitis, zystische Fibrose) wurden von der Studie ausgeschlossen.

Andere Einschluß-Kriterien waren: männliches und weibliches Geschlecht und Alter von mindestens 18 Jahren.

Weitere Ausschluß-Kriterien waren jede andere schwere Krankheit, die die Teilnahme an der Studie oder deren Vollendung eingeschränkt hätte (mit Ausnahme von Befunden, die der besagten Krankheit der Studie zugrundeliegen), bekannte Allergie auf Pankreatin und/oder Schweineinsulin, jede Art von Malignität im Zusammenhang mit dem Verdauungstrakt während der letzten 5 Jahre, jede Art von Magen-Darm-Operation und Kurzdarmsyndrom, Haemachromatose, jede Vorgeschichte von Drogenmißbrauch einschließlich Alkohol, positiver Urin-Schwangerschaftstest oder bestehende Schwangerschaft oder Stillzeit (bei Frauen), schwere Allergie oder jede Vorgeschichte von schweren abnormen Medikamentenreaktionen, vermutete Non-Compliance oder fehlende Kooperation, Einnahme jeglicher experimenteller Wirksubstanz in den letzten 4 Wochen vor Studienbeginn, und jeder andere Grund, der nach Meinung des Untersuchenden die Teilnahme des Patientens an der Studie verbieten würde.

### Design:

Alle Patienten wurden, nach Unterzeichnung des Informations/Zustimmungsformular, einem Vorscreening der Elastase-1 im Stuhl unterzogen. Sofern die Werte der Elastase-1 kleiner/gleich 100 ug/g Stuhl waren und die Patienten die Einschluß-Kriterien erfüllten und nicht von den Ausschlußkriterien betroffen waren, konnten sie in die Eingangsphase von mindestens 8 Wochen aufgenommen.

Während der Eingangsphase sollte der Patient eine stabile Insulindosierung erreichen, einschließlich keiner Veränderung der Anzahl der Insulin-Injektionen und des Insulintyps, keines Wechsels von Standardbehandlung zur Intensivbehandlung oder von Injektionen auf Pumpen. Der HbA_{1c}-Spiegel sollte im Bereich zwischen 0,07 und 0,10 (in metrischen Einheiten) liegen. Das Körpergewicht mußte stabil bleiben, mit Abweichungen von nicht mehr als 5 kg zwischen den Bewertungen.

Am Ende der Eingangsphase wurden geeignete Patienten zufallsverteilt, entweder auf Kreon® 10000 Minimicrospheres® oder auf Placebo. Die Behandlungsperiode wurde auf 16 Wochen angesetzt, mit mehreren Zwischenbewertungen nach 1, 2, 4 und 10 Wochen und einer Endbewertung nach 16 Wochen. Während der ersten 4 Wochen waren Insulin-Anpassungen gestattet. Nach 4, 10 und 16 Wochen wurden umfangreiche Untersuchungen zur glykämischen Kontrolle durchgeführt (siehe Studienziel).

### Behandlung und Dosierungen:

Die Patienten zufällig entweder dem Kreon® 10000 Minimicrospheres® oder dem Placebo zugewiesen.

Die Patienten sollten planmäßig 16 bis 18 Kapseln/Tag erhalten. Die Anzahl der Kapseln pro Mahlzeit war festgelegt. Zu den drei Hauptmahlzeiten sollten die Patienten 4 Kapseln und zu den (2 bis 3) Zwischenmahlzeiten 2 Kapseln einnehmen.

Dieses entspricht 40000 Ph. Eur. Lipase-Einheiten pro Haupt-Malhzeit und 20000 Ph. Eur. Lipase-Einheiten pro Zwischenmahlzeit. Eine gesamte Tagesdosis von 160000 bis 180000 Ph. Eur. Lipase-Einheiten wurde verabreicht. Dieses spiegelt die Dosis wider, die in klinischen Untersuchungen mit Patienten mit chronischer Pankreatitis angewendet wurde, und sollte daher für die Behandlung von Patienten mit exokriner Pankreasinsuffizienz ausreichend sein.

### Statistik:

Die Analyse der primären Parameter war eine Kovarianz-Analyse unter Verwendung eines linearen Modells mit der Kovariate als Basislinie, der fixierten Haupteffekte von Behandlung und Zentrum, der beachteten Maßnahmen über die Zeit, und der Behandlungs-Interaktion durch das Zentrum. Der alpha-Spiegel beim Testen betrug 5%. Die Analyse wurde in der zum Behandeln vorgesehenen Testgruppe durchgeführt, die aus allen zufallsverteilten Patienten besteht, die nach 4 Wochen Behandlung unter stabiler Insulindosis wenigstens eine HbA_{1c}-Messung vorwiesen. Da nachfolgend nur die erhaltene Zwischenanalyse berichtet wird, wurde keine Peer-Protokoll-Modellgruppe definiert.

Die Testgruppengröße wurde als 74 Patienten pro Gruppe gerechnet, unter Anwendung eines alpha-Spiegel von 5 %, einer Standardabweichung für HbA_{1c} von sigma = 0,015 und eine Stärke von 80 % um eine Differenz von 0,007 zu ermitteln.

Eine Zwischenanalyse ist vorgesehen, sobald die Hälfte der Patienten die Studie abgeschlossen haben (37 Patienten pro Gruppe).

### II. Ergebnisse der Studie

### Patientenguppe:

Wegen niedriger Rekrutierung wurden die Interims-Analyse mit 71 Patienten durchgeführt, die entweder auf Kreon® 10000 Minimicrospheres® oder Placebo zufallsverteilt waren. Die 71 Patienten sind in der Sicherheitstestgruppe eingeschlossen und haben wenigstens eine Dosis des Studienmedikamentens erhalten.

Basierend auf den Kriterien für die zum Behandeln vorgesehene Testgruppe (intent-to-treat sample, = ITT) konnten 65 Patienten für die HbA_{1c} Analyse (Tabelle 1) herangezogen werden.

**Tabelle 1**

| Patienten-Testgruppen | | | |
|---|---|---|---|
| | **Placebo** **N** | **Kreon** **N** | **Total** **N** |
| **Sicherheitstestgruppe** | 36 | 35 | 71 |
| **ITT-Testgruppe** | 36 | 29 | 65 |
| ITT = intent-to treat | | | |

### Demographische Daten:

Die nachfolgende Tabelle 2 gibt einen Überblick über die wichtigsten demographischen Daten.

**Tabelle 2 :**

| Demographischen Daten | | | |
|---|---|---|---|
| **Parameter** | **Placebo** | **Kreon** | **Total** |
| **Geschlecht [N(%)]** | | | |
| Männer Frauen | 21(58,3) 15(41,7) | 23(65,7) 12(34,3) | 44(62,0) 37(38,0) |

| **Ethnische Herkunft [N(%)]** | | | |
|---|---|---|---|
| Kaukasier Schwarze | 36(100,0) - | 34(97,1) 1(2,9) | 70(98,6) 1(1,4) |

| **Alter** (**Jahre)** | | | |
|---|---|---|---|
| Mittelwert ± SD Bandbreite Männer (Mittelwert ± SD) Frauen (Mittelwert ± SD) | 42,7±10,0 24-64 44,7±10,4 40,0±9,0 | 46,6±8,9 30-63 45,7±9,7 48,3±7,3 | 44,6±9,6 24-64 45,2±10,0 43,7±9,2 |

| **Gewicht (Kg)** | | | |
|---|---|---|---|
| Mittelwert ± SD Bandbreite Männer (Mittelwert ± SD) Frauen (Mittelwert ± SD) | 74,5±11,8 47-93 79,8±9,0 67,0±11,5 | 79,2±12,9 54-103 84,7±9,3 68,6±12,5 | 76,8±12,5 47-103 82,4±9,4 67,7±11,8 |

| **Größe (cm)** | | | |
|---|---|---|---|
| Mittelwert ± SD Bandbreite Männer (Mittelwert ± SD) Frauen (Mittelwert ± SD) | 171,1±10,1 152-190 176,3±7,4 163,8±8,7 | 173,2±10,0 156-191 179,1±6,4 161,8±3,6 | 172,1±10,0 152-191 177,8±7,0 162,9±6,9 |

| **BMI [Kg/m**^{**2**}**]** | | | |
|---|---|---|---|
| ± SD Mittelwert ± SD Bandbreite Männer (Mittelwert ± SD) Frauen (Mittelwert ± SD) | 25,4±3,0 18-31 25,7±2,6 24,9±3,6 | 26,4±3,6 19-34 26,5±3,2 26,1±4,3 | 25,9±3,3 18-34 26,1±3,0 25,5±3,9 |
| SD = Standard Deviation (Standardabweichung) | | | |
| BMI = Body Mass Index (Körpergewicht-Index) | | | |

Wie aus der Tabelle ersichtlich, sind die demographischen Daten der beiden Behandlungsgruppen vergleichbar, obgleich einige Männer mehr zur Kreon-Gruppe zufallsverteilt waren, was in der Folge zu leicht höheren Werten für Größe, Gewicht und Körpergewicht-Index in der Kreon-Gruppe führte. Jedoch können diese Abweichungen als unwesentlich für die Interpretation der Ergebnisse der Wirksamkeit angesehen werden.

Das Gleiche gilt für das Alter des Auftretens von Diabetes, die Dauer der Krankheit oder die Dauer der Insulinbehandlung. In allen Fällen gab es nur kleine Unterschiede in den Mitteloder Median-Werten zwischen den beiden Behandlungsgruppen. Daher kann die Schwere der Krankheit in den beiden Behandlungszweigen als ähnlich angesehen werden.

Die Elastase-1 Werte beim Vorscreening waren zwischen den zwei Gruppen ebenfalls nicht signifikant verschieden (57,3 ± 26,5 für Kreon gegen 62,0 ± 29,8 für Placebo).

### Parameter der Wirksamkeit:

Die Ergebnisse für die wichtigsten Parameter des HbA_{1c} sind in Tabelle 3 zusammengefaßt. Die Tabelle enthält die Daten der ITT-Testgruppe.

**Tabelle 3:**

| HbA_{1c} - ITT-Testgruppe | | | | |
|---|---|---|---|---|
| | **Placebo** | | **Kreon** | |
| | **N** | **Mittelwert** | **N** | **Mittelwert** |
| **4. Woche** | 36 | 0,07967 | 29 | 0,07779 |
| Unterschiede zwischen den Behandlungen | Mittelwert: 0,00187 SEM: 0,00086 P=0,0330 | | | |

| **10. Woche** | 36 | 0,08131 | 29 | 0,07876 |
|---|---|---|---|---|
| Unterschiede zwischen den Behandlungen | Mittelwert: 0,00254 SEM: 0,00112 P=0,0268 | | | |

| **16. Woche** | 36 | 0,08273 3 | 29 | 0,08034 |
|---|---|---|---|---|
| Unterschiede zwischen den Behandlungen | Mittelwert: 0,00238 SEM: 0,00138 P=0,0902 | | | |
| SEM = Standard Error of the Mean (Mittlerer Standardfehler) | | | | |

Die Tabelle 3 zeigt, daß Kreon zu statistisch siginifikant niedrigeren HbA_{1c}-Spiegeln nach 4 und 10 Wochen führt, und zu einem klaren Trend sogar nach 16 Wochen. Dieses zeigt, daß Kreon das Potential zur Verbesserung der glykämischen Kontrolle bei Patienten mit Insulin-abhängigem Diabetes mellitus besitzt.

Dieses wird durch Tabelle 4 unterstrichen, welche die Anzahl der Patienten mit Verbesserung, ohne Veränderung oder mit Verschlechterung bezogen auf die HbA_{1c}-Werte der Basislinie angibt.

**Tabelle 4:**

| | | | | | | |
|---|---|---|---|---|---|---|
| Anzahl der Patienten mit Verbesserung, ohne Veränderung oder mit Verschlechterung bezogen auf die HbA_{1c}-Werte der Basislinie | | | | | | |

| | **Placebo** | | | **Kreon** | | |
|---|---|---|---|---|---|---|
| | **verbessert** | **unverändert** | **verschlechtert** | **verbessert** | **unverändert** | **verschlechtert** |
| **4. Woche** | 13 | 5 | 18 | 15 | 5 | 9 |
| **10. Woche** | 12 | 2 | 22 | 12 | 5 | 12 |
| **16. Woche** | 4 | 4 | 26 | 11 | 3 | 15 |

Unter Placebo-Behandlung zeigten viel mehr Patienten eine Verschlechterung ihrer HbA_{1c}-Spiegel, über den zeitlichen Vergleich mit Kreon. Die Tendenz zur Verschlechterung ist relativ gering unter Kreon, wohingegen unter Placebo die Mehrheit der Patienten Verschlechterung erfuhren. Wiederum ein klares Zeichen, daß Kreon effektiv in der Lage ist, die glykämische Kontrolle des Insulin-abhängigen Diabetes mellitus zu verbessern. Der Grund, weshalb viele Patienten sich verschlechterten liegt im Studiendesign, das eine beinahe optimale Behandlung vor der Zufallsverteilung verlangt, was unter Normalbedingung, wie bei der Placebo-Gruppe zu sehen war, zu einem schenellen Verfall der glykämischen Kontrolle führt. Kreon ist in der Lage, diese Verschlechterung zu verringern. Ähnliche Befunde wurden beobachtet bezüglich der Anzahl der aufgetretenen milden bis mäßigen hyperglykämischen Attacken (Tabelle 5).

**Tabelle 5:**

| Veränderung der Anzahl hyperglykämischer Attacken | | | | | | |
|---|---|---|---|---|---|---|
| | **Placebo** | | | **Kreon** | | |
| | **<0** | **=0** | **>0** | **<0** | **=0** | **>0** |
| **4. Woche** | 11 | 9 | 16 | 8 | 7 | 14 |
| **10. Woche** | 13 | 13 | 10 | 13 | 3 | 11 |
| **16. Woche** | 14 | 10 | 12 | 14 | 8 | 8 |

| | | | | | | |
|---|---|---|---|---|---|---|
| <0 = weniger Attacken im Vergleich zur Basislinie | | | | | | |
| =0 = gleiche Anzahl von Attacken im Vergleich zur Basislinie | | | | | | |
| >0 = größere Anzahl von Attacken im Vergleich zur Basislinie | | | | | | |

Die Tabelle 5 zeigt, daß beinahe 50 % der Patienten, die Kreon erhielten, weniger milde bis moderate hyperglykämische Attacken hatten, verglichen mit der Placebo-Gruppe, in der nur 39 % der Patienten diesen Befund hatten.

In bezug auf die Anzahl erhöhter Glukosespiegel pro Woche - ein Indikator für hyperglykämischen Perioden - wurde ein vergleichbarer, wenn auch weniger starker Effekt gefunden.

Nüchterne und nicht-nüchterne Blut-Glukosespiegel waren nicht signifikant verschieden, ebenso wie die von den Patienten benötigte Insulindosis.

Die Anzahl negativer Vorfälle war bei den beiden Behandlungsgruppen nicht verschieden. Bei der Behandlung mit Kreon wurden im Verdauungssystem einige negative Vorfälle mehr beobachtet, und unter Placebobehandlung mehr negative Vorfälle in der metabolischen/Ernährungs-Störungs- sowie der Atmungssystem-Gruppe negativer Vorfälle. Die Anzahl der Patienten, die die wegen negativer Vorfälle aufgaben, war bei beiden Gruppen gleich.

### III. Schlußfolgerung

Zusammenfassend, gibt es starke Anhaltspunkte dafür, daß Kreon sich günstig auf die Behandlung von Insulin-abhängigen diabetischen Patienten mit exokriner Pankreasinsuffizienz auswirkt und zu einer besseren glykämischen Kontrolle führt. Dieses bedeutet, daß Kreon bei der Behandlung dieser Patienten hinzugegeben werden sollte, um den wichtigsten Faktor in deren Behandlung zu verbessern.

Das nachfolgende Beispiel soll die Herstellung einer Pankreatin enthaltenden, zur Diabetes-Behandlung, insbesondere zur adjuvanten Diabetes-Behandlung, geeigneten pharmazeutischen Zubereitung näher erläutern, ohne jedoch den Umfang der Erfindung zu beschränken.

### Beispiel

120 kg Pankreatin wurden in einem handelsüblichen Mischer mit 30 kg Polyethylenglykol 4000 gemischt und mit ca. 20 kg Propan-2-ol durchfeuchtet.

Die Mischung wurde durch eine Strangpresse (Extruder), welche mit einer Lochmatritze mit Bohrungen von 0,8 mm lichter Weite und einer dahinterliegenden Schneidevorrichtung ausgestattet war, gedrückt. Hierbei wurden Strangbruchstücke mit einer Stranglänge von bis zu 20 mm erhalten.

In Portionen von je ca. 15 kg wurden die Strangbruchstücke in einem Rundungsgerät (Typ Caleva) zerbrochen und zu sphärisch geformten Pellets ausgerundet, wobei man jeder Portion noch 300 g dünnflüssiges Paraffin und abhängig von der Verweilzeit im Rundungsgerät (3 bis 6 Min.) noch ca. 300 bis 700 g Propan-2-ol zufügte.

Nach der Trocknung in einem handelsüblichen Hordenblechtrockner erhielt man als Ausbeute ca. 90 % an Pankreatinmikropelletkernen mit einem Durchmesser von 0,7 bis 1,4 mm, klassiert mit einem 0,7 mm-Sieb (Absiebung von Unterkorn < 0,7 mm) und einem 1,4 mm-Sieb (Absiebung von Überkorn > 1,4 mm), mit einem Pankreatingehalt von ca. 78 %. Die Schüttdichte betrug 0,7 g/ml.

Anschließend wurden die Mikropelletkerne in einer üblichen Befilmungsapparatur mit einer Lösung aus Hydroxypropylmethylcellulosephtalat (Typ HP55), Dibutylphtalat, dünnflüssigem Paraffin und Silikonöl (Dimethicone 1000) in Aceton auf an sich bekannte Weise magensaftresistent überzogen. Als Ausbeute erhielt man ca. 90 % magensaftresistente Pankreatinmikropellets, mit einem Durchmesser im Bereich von 0,7 bis 1,6 mm, klassiert mit einem 0,7 mm-Sieb (Absiebung von Unterkorn < 0,7 mm) und einem 1,6 mm-Sieb (Absiebung von Überkorn > 1,6 mm) mit einem Gehalt von ca. 60 % Pankreatin, bezogen auf die filmüberzogenen Mikropellets, und einer Schüttdichte von 0,8 g/ml.

Anschließend wurden die Mikropellets in handelsübliche Hartgelatinekapseln oder Sachets abgefüllt.

## Patentansprüche

1. Verwendung von physiologisch akzeptablen Enzymgemischen mit lipolytischer, proteolytischer und amylolytischer Aktivität zur Herstellung von pharmazeutischen Zubereitungen zur Behandlung von primären Diabetes mellitus Typ I in größeren Säugetieren oder Menschen.

2. Verwendung nach Anspruch 1, **dadurch gekennzeichnet, dass** man als Enzymgemisch ein Gemisch aus mikrobiell gewonnenen Lipasen, Proteasen und Amylasen verwendet.

3. Verwendung nach Anspruch 1, **dadurch gekennzeichnet, dass** man als Enzymgemisch Pankreatin und/oder ein Pankreatin-ähnliches Verdauungsenzymgemisch verwendet.

4. Verwendung nach Anspruch 3, **dadurch gekennzeichnet, dass** man als Pankreatin-ähnliches Verdauungsenzymgemisch ein pankreatinhaltiges Verdauungsenzymgemisch verwendet.

5. Verwendung nach Anspruch 3 oder 4, **dadurch gekennzeichnet, dass** man Pankreatin oder ein pankreatinhaltiges Verdauungsenzymgemisch verwendet, wobei dem Pankreatin oder dem pankreatinhaltigen Verdauungsenzymgemisch zusätzlich noch ein oder mehrere mikrobielle Enzyme aus der Gruppe der Lipasen, Proteasen und Amylasen zugesetzt sind.

6. Verwendung nach einem der Ansprüche 1 bis 5, **dadurch gekennzeichnet, dass** man pharmazeutische Zubereitungen zur adjuvanten Behandlung von Diabetes mellitus herstellt.

7. Verwendung nach einem der Ansprüche 1 bis 6, **dadurch gekennzeichnet, dass** man pharmazeutische Zubereitungen zur Behandlung von mit exokriner Pankreasinsuffizienz begleitetem Diabetes mellitus herstellt.

8. Verfahren zur Herstellung von pharmazeutischen Zubereitungen zur Behandlung von primärem Diabetes mellitus Typ I, **dadurch gekennzeichnet, dass** man eine klinische diabetische Parameter verbessernd wirksame und/oder eine die Spätfolgen des Diabetes vermindernd wirksame Menge eines physiologisch akzeptablen Enzymgemisches mit lipolytischer, proteolytischer und amylolytischer Aktivität, vorzugsweise Pankreatin und/oder ein Pankreatin-ähnliches Verdauungsenzymgemisch, zusammen mit üblichen pharmazeutischen Hilfsstoffen in eine geeignete Arzneiform überführt.

## Claims

1. Use of physiologically acceptable enzyme mixtures with lipolytic, proteolytic and amylolytic activity for the manufacture of pharmaceutical preparations for the treatment of type I primary diabetes mellitus in larger mammals or humans.

2. Use according to Claim 1, **characterised in that** a mixture of microbially synthesised lipases, proteases and amylases is used as enzyme mixture.

3. Use according to Claim 1, **characterised in that** pancreatin and/or a pancreatin-like mixture of digestive enzymes is used as enzyme mixture.

4. Use according to Claim 3, **characterised in that** a mixture of digestive enzymes containing pancreatin is used as a pancreatin-like mixture of digestive enzymes.

5. Use according to Claim 3 or 4, **characterised in that** pancreatin or a mixture of digestive enzymes containing pancreatin is used, one or more microbial enzymes from the group of lipases, proteases and amylases being additionally added to the pancreatin or the mixture of digestive enzymes containing pancreatin.

6. Use according to one of Claims 1 to 5, **characterised in that** pharmaceutical preparations for the adjuvant treatment of diabetes mellitus are manufactured.

7. Use according to one of Claims 1 to 6, **characterised in that** pharmaceutical preparations for the treatment of diabetes mellitus accompanied by exocrine pancreatic insufficiency are manufactured.

8. Process for the manufacture of pharmaceutical preparations for the treatment of type I primary diabetes mellitus, **characterised in that** a quantity of a physiologically acceptable enzyme mixture with lipolytic, proteolytic and amylolytic activity, preferably pancreatin and/or a pancreatin-like mixture of digestive enzymes, that is effective in improving the clinical parameters of diabetes and/or effective in reducing the late complications of diabetes together with conventional pharmaceutical auxiliaries is converted into a suitable dosage form.

## Revendications

1. Utilisation de mélanges enzymatiques physiologiquement acceptables à activité lipolytique, protéolytique et amylolytique pour la fabrication de préparations pharmaceutiques pour le traitement du diabète sucré primaire de type I chez les mammifères supérieurs ou chez l'homme.

2. Utilisation selon la revendication 1, **caractérisée en ce qu'**on utilise comme mélange enzymatique un mélange de lipases, de protéases et d'amylases obtenues par voie microbienne.

3. Utilisation selon la revendication 1, **caractérisée en ce qu'**on utilise comme mélange enzymatique de la pancréatine et/ou un mélange enzymatique de digestion analogue à la pancréatine.

4. Utilisation selon la revendication 3, **caractérisée en ce qu'**on utilise comme mélange enzymatique de digestion analogue à la pancréatine un mélange enzymatique de digestion contenant de la pancréatine.

5. Utilisation selon la revendication 3 ou 4, **caractérisée en ce qu'**on utilise de la pancréatine ou un mélange enzymatique de digestion contenant de la pancréatine, en ajoutant en outre à la pancréatine ou au mélange enzymatique de digestion contenant de la pancréatine, un ou plusieurs enzymes microbiens du groupe des lipases, des protéases et des amylases.

6. Utilisation selon l'une des revendications 1 à 5, **caractérisée en ce qu'**on fabrique des préparations pharmaceutiques pour le traitement auxiliaire du diabète sucré.

7. Utilisation selon l'une des revendications 1 à 6, **caractérisée en ce qu'**on fabrique des préparations pharmaceutiques pour le traitement du diabète sucré accompagné d'insuffisance du pancréas exocrine.

8. Procédé de fabrication de préparations pharmaceutiques pour le traitement du diabète sucré primaire du type I, **caractérisé en ce qu'**on met une quantité efficace - améliorant un paramètre diabétique clinique - et/ou une quantité efficace - réduisant les conséquences du diabète - d'un mélange enzymatique physiologiquement acceptable à activité lipolytique, protéolytique et amylolytique, de préférence de la pancréatine et/ou un mélange enzymatique de digestion analogue à la pancréatine, conjointement avec des substances auxiliaires pharmaceutiques usuelles, sous une forme pharmaceutique appropriée.
